Europäisches Patentamt

European Patent Office

Office européen des brevets

(19)

(11) Publication number: **0 161 931**
**B1**

# EUROPEAN PATENT SPECIFICATION

(12)

(45) Date of publication of patent specification: **03.05.89**

(51) Int. Cl.⁴: **G 01 N 33/00**

(21) Application number: **85303364.5**

(22) Date of filing: **13.05.85**

(54) **Gas analyzer.**

(30) Priority: **14.05.84 US 609673**

(43) Date of publication of application:
**21.11.85 Bulletin 85/47**

(45) Publication of the grant of the patent:
**03.05.89 Bulletin 89/18**

(84) Designated Contracting States:
**AT BE DE FR GB IT NL SE**

(56) References cited:
**DE-B-2 912 181**
**US-A-4 094 187**
**US-A-4 322 964**

(73) Proprietor: **THE BABCOCK & WILCOX COMPANY**
**1010 Common Street P.O. Box 60035**
**New Orleans Louisiana 70160 (US)**

(72) Inventor: **Barnett, Daniel C.**
**10100 Chipmunk Ridge**
**Concord Township Ohio 44077 (US)**

(74) Representative: **Purvis, William Michael Cameron et al**
**D. Young & Co. 10 Staple Inn**
**London WC1V 7RD (GB)**

Courier Press, Leamington Spa, England.

## Description

The invention relates to gas analyzers for analyzing the presence and/or quantity of a gas and particularly to a method and apparatus for calibrating gas analyzers using a test gas of known properties.

Gas analyzers are known which use aspirators that function to draw a sample gas from an environment, for example from the interior of a gas duct, and conduct that sample to a sensor or other sampling system which performs an analytical test on the sample.

The sample gas is drawn by a probe to the sensor or sampling system. Such probes usually extend into the environment to be tested and are used in conjunction with a filter for filtering out debris.

Such gas analyzers are known to require some mechanism for applying a test gas in order accurately to check the calibration of the instrument. Simply flooding the system with the test gas to overcome the sample gas entering the probe is not an accurate check since this pressurizes the system which is normally operated at negative pressure.

Such a method also cannot check for the existence of leaks in the normally negatively pressurized system.

Such a method also produces more flow rate through the sensor cavities which will represent an error particularly in certain types of sensors which are sensitive to flow rate. This would also represent an error in sensors which utilize a test gas that is diluted with air at constant flow, for example in the case of carbon monoxide sensors which require dilution air for combustion of the catalyst.

Prior art gas analyzers are shown in US—A—4 322 964, US—A—4 094 187 and DE—A—2 912 181.

Other methods of calibrating a gas analyzer include physically blocking the probe to prevent entry of the sample gas and to prevent mixing between the sample gas and the test gas. This is usually accomplished by physically inserting a plug into the probe or into a connecting passage for the probe. The test gas is then injected into a port in the sampling or analyzing system which is downstream of the mechanical plug. Shortcomings of this method include frequent failures of seals used to block off the same probe or failures in seals associated with shafts used for moving the plug assembly for plugging the probe. Other problems include seizing between two relatively movable parts where metal to metal thread seals are utilized. This is due to the high temperatures required by some sampling systems, as well as high temperatures which may exist in the duct or environment containing the sample gas. In addition to the foregoing problems, the prior art methods require a local calibration unless a solenoid shut-off is used which is also affected by high temperatures and requires seals.

According to one aspect of the invention there is provided a method of analyzing a sample gas from a test space using a gas analyzer wherein:

the sample gas to be analyzed is drawn at a selected flow rate through the gas analyzer from a probe extending into the test space and through an input line connected between the probe and the analyzer and is discharged from the analyzer through an output line by powering a sample aspirator connected to the output line; characterised in that, to calibrate the analyzer, a test gas aspirator in the input line is powered to equalise the flow in the output line with a reverse flow in the input line whereby the supply of sample gas to the analyzer is stopped; and

a test gas to be used in calibrating the analyzer is supplied at the selected flow rate to the analyzer through the input line and is discharged over the output line.

According to another aspect of the invention there is provided apparatus for analyzing a sample gas from a test space, comprising:

a probe extending into the test space;

an input line connected to the probe;

a gas analyzer connected to the input line to receive gas to be sampled from the input line;

an output line to discharge gas from the analyzer; and

sample aspiration means connected to the output line and comprising an aspirator having a power input connected to a source of pressure and a sample input connected to the output line to draw sample gas at a selected rate from the test space through the probe, the input line, the analyzer and the output line; characterised by

test gas aspiration means connected to the input line and comprising an aspirator having a power line for receiving a source of pressure and connected to the input line, the power line having a valve therein for adjusting the power of the test gas aspirator to stop flow of gas through the analyzer by equalizing flow of gas in the output line with a reverse flow of gas in the input line;

flow measuring means connected to the input line for measuring flow in the input line; and

test gas supply means connected to the input line to supply test gas to the input line at the selected flow rate for calibration of the analyzer.

Thus a reverse aspiration in the form of the test gas aspiration means is utilized to block the flow of sample gas through the analyzer without the use of mechanical moving parts and simultaneously the test gas is supplied to the analyzer at the same pressure and flow rate at which the sample gas was supplied, thereby avoiding problems associated with methods utilizing pressurized test gas.

The absence of moving parts or seals and lack of any mechanical probe shut-off devices which could be adversely affected by high ambient and operating temperatures can be a considerable advantage.

The invention is diagrammatically illustrated by way of example with reference to the accompanying drawings, in which:—

Figure 1 is a schematic block diagram showing apparatus according to the invention in operation to analyze a sample gas;

Figure 2 is a view similar to Figure 1 showing the apparatus in a position which is preparatory for a calibration of the gas analyzer; and

Figure 3 is a view similar to Figure 1 of the apparatus in a position for calibrating the gas analyzer.

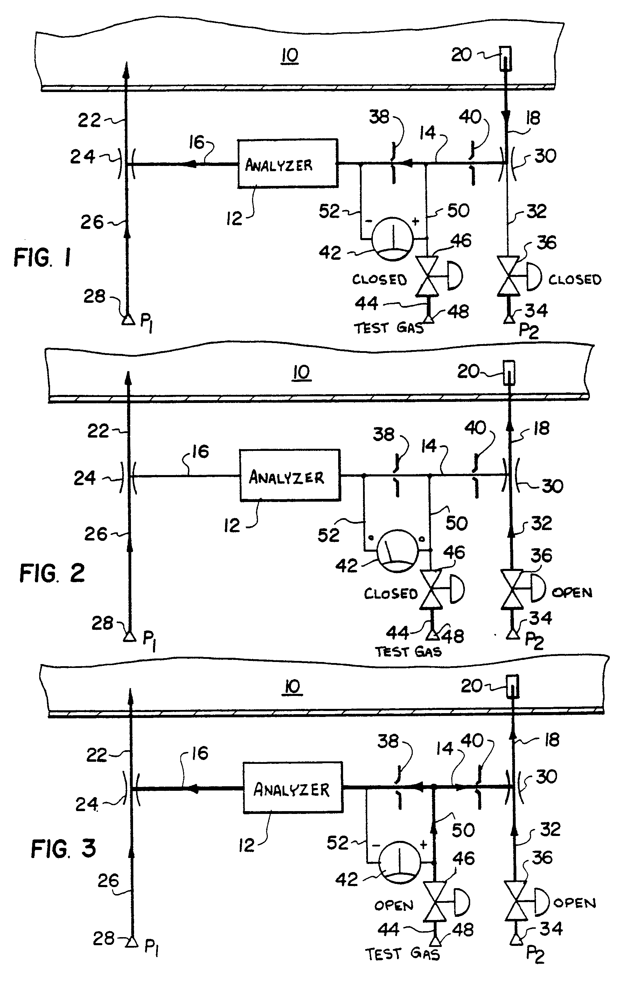

Referring to the drawings and firstly to Figure 1, gas analyzer apparatus is shown for analyzing a sample gas from a test space 10 which may be the interior of a process vessel or a conduit for carrying a gas sample, such as exhaust from a combustion process. The arrangement utilizes a gas analyzer 12 which is of known design and which includes an input line 14 for supplying a sample gas to a sensor and an output line 16 for discharging the sample gas from the sensor.

A probe 18, which is also of known design, has an open input end which is covered by a filter 20 and extends into the test space 10. A return line 22 is connected between the output line 16 and the test space 10 for returning the tested sample gas to the test space.

A sample aspirator 24, which itself is of known design, has a power input line 26 connected to a source of pressure 28 which is at level $P_1$. An orifice of the sample aspirator 24 is connected to the output line 16 so that, depending on the pressure $P_1$ in the power line 26, a selected flow rate will be established on the output line 16. The sample gas as well as the powering gas is discharged over the line 22 into the test space 10. The selected flow rate also induces a flow of sample gas, at the same flow rate, over the input line 14, which draws sample gas from the test space 10, through the filter 20 and the probe 18.

A test gas aspirator 30 is connected between the probe 18 and the input line 14. The aspirator 30 is of similar design to the aspirator 24 and is powered by a second power line 32 connected to a second source of pressure 34 which provides a pressure $P_2$. The power line 32 can be isolated from the source 34 by a power line valve 36.

The input line 14 is provided with a first orifice 38 and a second orifice 40. A pressure gauge 42 for measuring the pressure drop across the orifice 38 is connected to the input line 14 upstream and downstream of the first orifice 38. This pressure drop across the orifice 38 is proportional to the selected flow rate and thus can be utilized as a measurement for the flow rate.

Connected to the upstream side of the pressure gauge 42 is a test gas line 44 which includes a test gas valve 46 and is connected to a source of test gas at 48.

Figure 1 illustrates the flow of sample gas through the analyzer 12 for analyzing the sample gas. In this measuring condition for the analyzer arrangement, the power line valve 36 and the test gas valve 46 are closed. The flow rate as measured by the pressure gauge 42 is determined by the pressure $P_1$ on the power line 26 through the aspirator 24.

Figure 2 shows a flow condition when the analyzer 12 is being prepared for calibration. In this condition, the valve 36 is open unitl the aspirator 30, powered by the pressure $P_2$ is drawing gas from the input line 14 at the same rate as the aspirator 24 is drawing gas from the output line 16. The pressures in the input 14 and output 16 lines are equalized and no gas flows through the analyzer 12. The pressure drop as indicated by the pressure gauge 42 falls to zero since there is no flow in the input line 14 and thus no pressure drop across the orifice 38.

At this stage, as illustrated in Figure 3, the test gas valve 46 is opened by a selected amount and supplies test gas from the source 48 over an upstream line 50 to the input line 14. The test gas flows in both directions over the orifices 38 and 40. The valve 46 is held open until the flow rate, indicated on the pressure gauge 42, is equal to the selected flow rate for the sample gas illustrated in Figure 1. The test gas is thus supplied to the analyzer 12 under the same conditions as the sample gas was provided to the analyzer so that unambiguous calibration can take place.

The test gas also flows in the reverse direction on the line 14 through the aspirator 30 back to the test space 10 but this does not adversely affect the calibration step.

## Claims

1. A method of analyzing a sample gas from a test space (10) using a gas analyzer (12) wherein:

the sample gas to be analyzed is drawn at a selected flow rate through the gas analyzer (12) from a probe (18) extending into the test space (10) and through an input line (14) connected between the probe (18) and the analyzer (12) and is discharged from the analyzer (12) through an output line (16) by powering a sample aspirator (24) connected to the outut line (16); characterised in that, to calibrate the analyzer (12), a test gas aspirator (30) in the input line (14) is powered to equalise the flow in the output line (16) with a reverse flow in the input line (14) whereby the supply of sample gas to the analyzer (12) is stopped; and

a test gas (48) to be used in calibrating the analyzer (12) is supplied at the selected flow rate to the analyzer (12) through the input line (14) and is discharged over the output line (16).

2. A method according to claim 1, wherein a pressure drop across a first orifice (38) provided in the input line (14) is measured to determine the selected flow rate for the sample gas, the test gas aspirator (30) is powered until the pressure drop across the first orifice (38) drops to zero and the test gas is then supplied to the input line (14) upstream of the first orifice (38) at a rate to return the pressure drop across the first orifice (38) to a level corresponding to the selected flow rate.

3. Apparatus for analyzing a sample gas from a test space (10), comprising:

a probe (18) extending into the test space (10);

an input line (14) connected to the probe (18);

a gas analyzer (12) connected to the input line (14) to receive gas to be sampled from the input line (14);

an output line (16) to discharge gas from the analyzer (12); and

sample aspiration means (24) connected to the output line (16) and comprising an aspirator having a power input connected to a source of pressure $(P_1)$ and a sample input connected to the output line (16) to draw sample gas at a selected rate from the test space (10) through the probe (18), the input line (14), the analyzer (12) and the output line (16); characterised by

test gas aspiration means (30) connected to the input line (14) and comprising an aspirator having a power line (32) for receiving a source of pressure $(P_2)$ and connected to the input line (14), the power line (32) having a valve (36) therein for adjusting the power of the test gas aspirator (30) to stop flow of gas through the analyzer (12) by equalizing flow of gas in the output line (16) with a reverse flow of gas in the input line (14);

flow measuring means (38, 42) connected to the input line (14) for measuring flow in the input line (14); and

test gas supply means (48) connected to the input line (14) to supply test gas to the input line (14) at the selected flow rate for calibration of the analyzer (12).

4. Apparatus according to claim 3, wherein the flow measuring means comprises a first orifice (38) connected in the input line (14) and a pressure gauge (42) connected between an upstream side and a downstream side of the first orifice (38) and to the input line (14), the test gas supply means (48) being connected to the upstream side of the first orifice (38).

5. Apparatus according to claim 4, including a second orifice (40) in the input line (14) upstream of the first orifice (38) and upstream of the pressure gauge (42).

## Patentansprüche

1. Verfahren zum Analysieren eines Probegases aus einem Testraum (10) unter Verwendung eines Gasanalysators (12), wobei das zu analysierende Probegas mit einer gewählten Flußrate aus einer Prüfsonde (18), welche sich in den Testraum (10) hinein erstreckt durch den Gasanalysator (12) und durch einen Einlaßleitung (14) hindurch gezogen wird, welche zwischen der Prüfsonde (18) und dem Analysator (12) angeschlossen ist, und aus dem Analysator (12) durch eine Ausgangsleitung (16) ausgegeben wird, in dem ein an die Auslaßleitung (16) angeschlossener Probensaugapparat (24) mit Energie versorgt wird, dadurch gekennzeichnet, daß zum Kalibrieren des Analysators (12) ein Testgassaugapparat (30) in der Eingangsleitung (14) mit Energie versorgt wird, um den Fluß in der Ausgangsleitung (16) auszugleichen durch einen umgekehrten Fluß in der Einlaßleitung (14), wodurch die Zufuhr von Probegas zu dem Analysator (12) gestoppt wird; und

daß ein Testgas (48), welches für die Kalibrierung des Analysators (12) verwendet werden soll, mit der gewählten Flußrate dem Analysator durch die Einlaßleitung (14) zugeführt und über die Ausgangsleitung (16) abgegeben wird.

2. Verfahren nach Anspruch 1, wobei ein Druckabfall über einer ersten Lochblende (38), welche in der Einlaßleitung (14) vorgesehen ist, gemessen wird, um die gewählte Flußrate für das Probegas zu bestimmen, der Testgassaugapparat (30) mit Energie versorgt wird, bis der Druckabfall über der erstsn Lochblende (38) auf Null abfällt und daß Testgas dann der Einlaßleitung (14) stromaufwärts von der ersten Lochblende (38) mit einer Rate zugeführt wird, derart, daß der Druckabfall über der ersten Lochblende (38) auf einen Wert zurückkehrt, welcher der gewählten Flußrate entspricht.

3. Vorrichtung zum Analysieren eines Probegases aus einem Testraum (10) mit:

einer Prüfsonde (18), welche sich in den Testraum (10) hinein erstreckt;

einer Einlaßleitung (14), welche mit der Prüfsonde (18) verbunden ist;

einem Gasanalysator (12), welcher an die Einlaßleitung (14) angeschlossen ist, um Gas aufzunehmen, welches probeweise aus der Einlaßleitung (14) genommen wird;

einer Auslaßleitung (16) zum Ausstoßen von Gas aus dem Analysator (12); und

einer Probensaugeinrichtung (24), welche an die Auslaßleitung (16) angeschlossen ist und einen Saugapparat aufweist, welcher eine Energiezufuhreinrichtung hat, die an eine Druckquelle $(P_1)$ angeschlossen ist, sowie einen Probeneingang, welcher an die Ausgangsleitung (16) angeschlossen ist, um Probegas mit einer gewählten Rate aus dem Testraum (10) durch die Prüfsonde (18), die Einlaßleitung (14), den Analysator (12) und die Auslaßleitung (16) zu ziehen; gekennzeichnet durch:

eine Testgassaugeinrichtung (30), welche mit der Einlaßleitung (14) verbunden ist und einen Saugapparat aufweist, der eine Leistungsleitung (32) hat zur Aufnahme einer Druckquelle $(P_2)$ und mit der Einlaßleitung (14) verbunden ist, wobei in der Leistungsleitung (32) ein Ventil (36) zum Einstellen der Leistung des Testgassaugapparrates (30) ist, um den Gasfluß durch den Analysator (12) durch Ausgleichen des Gasflusses in der Auslaßleitung (16) mit einem umgekehrten Fluß von Gas in der Einlaßleitung (14) zu stoppen;

eine Flußmeßeinrichtung (38, 42), welche an die Einlaßleitung (14) zum Messen des Flußes in der Einlaßleitung (14) angeschlossen ist; und

eine Testgaszufuhreinrichtung (48), welche an die Einlaßleitung (14) angeschlossen ist, um der Einlaßleitung (14) Testgas mit der gewählten Flußrate zum Kalibrieren des Analysators (12) zuzuführen.

4. Vorrichtung nach Anspruch 3, wobei die Flußmeßeinrichtung eine erste Lochblende (38) aufweist, welche in der Einlaßleitung (14) angeschlossen ist, und ein Druckmeßgerät (42), welches zwischen einer stromaufwärtigen Seite und einer stromabwärtigen Seite der ersten Lochblende (38) an die Einlaßleitung (14) angeschlossen ist, wobei die Testgaszufuhreinrichtung (48) an die stromaufwärtige Seite der ersten Lochblende (38) angeschlossen ist.

5. Vorrichtung nach Anspruch 4, welcher eine zweite Lochblende (40) in der Einlaßleitung (14) stromaufwärts von der ersten Lochblende (38) und stromaufwärts von dem Druckmeßgerät (42) aufweist.

## Revendications

1. Procédé d'analyse d'un gaz échantillon provenant d'un espace d'essai (10) en utilisant un analyseur de gaz (12) dans lequel:

le gaz échantillon à analyser est aspiré à un débit choisi à travers l'analyseur de gaz (12) depuis une sonde (18) s'étendant dans l'espace d'essai (10) et à travers une canalisation d'entrée (14) montée entre la sonde (18) et l'analyseur (12) et déchargé depuis l'analyseur (12) à travers une canalisation de sortie (16) en alimentant un aspirateur d'échantillon (24) relié à la canalisation de sortie (16); caractérisé en ce que, en vue d'étalonner l'analyseur (12), un aspirateur de gaz d'essai (30) dans la canalisation d'entrée (14) est alimenté pour égaliser l'écoulement dans la canalisation de sortie (16) avec un écoulement inverse dans la canalisation d'entrée (14) de sorte que la délivrance de gaz échantillon à, l'analyseur (12) est stoppée; et un gaz d'essai (48) à utiliser pour étalonner l'analyseur (12) est délivré à un débit choisi à l'analyseur (12) par l'intermédiaire de la canalisation d'entrée (14) et est déchargé par l'intermédiaire de la canalisation de sortie (16).

2. Procédé selon la revendication 1, dans lequel une chute de pression à travers un premier orifice (38) prévu dans la canalisation d'entrée (14) est mesurée pour déterminer le débit choisi pour le gaz échantillon, l'aspirateur de gaz d'essai (30) est alimenté jusqu'à ce que la chute de pression à travers le premier orifice (38) tombe à zéro et le gaz d'essai est ensuite délivré à la canalisation d'entrée (14) en amont du premier orifice (38) à un débit en vue de ramener la chute de pression à travers le premier orifice (38) à un niveau correspondant au débit choisi.

3. Appareil en vue d'analyser un gaz échantillon provenant d'un espace d'essai (10), comportant:

une sonde (18) s'étendant dans l'espace d'essai (10);

une canalisation d'entrée (14) reliée à la sonde (18); un analyseur de gaz (12) relié à la canalisation d'entrée (14) pour recevoir un gaz à échantillonner provenant de la canalisation d'entrée (14);

une canalisation de sortie (16) pour décharger le gaz provenant de l'analyseur (12); et

des moyens d'aspiration d'échantillon (24) reliés à la canalisation de sortie (16) et comportant un aspirateur possédant une entrée d'alimentation reliée à une source de pression ($P_1$) et une entrée d'échantillon reliée à la canalisation de sortie (16) pour aspirer un gaz échantillon à un débit choisi en provenance de l'espace d'essai (10) par l'intermédiaire de la sonde (18), la canalisation d'entrée (14), l'analyseur (12) et la canalisation de sortie (16); caractérisé par

des moyens (30) d'aspiration de gaz d'essai reliés à la canalisation d'entrée (14) et comportant un aspirateur possédant une canalisation d'alimentation (32) pour recevoir une source de pression ($P_2$) et relié à la canalisation d'entrée (14), la canalisation d'alimentation (32) possédant une vanne (36) disposée à l'intérieur en vue de régler l'alimentation de l'aspirateur (30) de gaz d'essai pour arrêter un écoulement de gaz à travers l'analyseur (12) en égalisant un écoulement de gaz dans la canalisation de sortie (16) avec un écoulement inverse de gaz dans la canalisation d'entrée (14);

des moyens (38, 42) de mesure d'écoulement reliés à la canalisation d'entrée (14) en vue de mesurer un écoulement dans la canalisation d'entrée (14); et

des moyens (48) d'alimentation en gaz d'essai reliés à la canalisation d'entrée (14) pour délivrer un gaz d'essai à la canalisation d'entrée (14) au débit choisi en vue d'un étalonnage de l'analyseur (12).

4. Appareil selon la revendication 3, dans lequel les moyens de mesure d'écoulement comportent un premier orifice (38) relié à la canalisation d'entrée (14) et une jauge de pression (42) montée entre un côté amont et un côté aval du premier orifice (38) et à la canalisation d'entrée (14), les moyens (48) d'alimentation en gaz d'essai étant reliés au côté amont du premier orifice (38).

5. Appareil selon la revendication 4, comprenant un second orifice (40) dans la canalisation d'entrée (14) en amont du premier orifice (38) et en amont de la jauge de pression (42).

EP 0 161 931 B1

FIG. 1

FIG. 2

FIG. 3

1